# EUROPEAN PATENT APPLICATION

(11) **EP 1 548 443 A1**
(43) Date of publication of application: **29.06.2005**
(21) Application number: 03784517.9
(22) Date of filing: 04.08.2003
(51) Int. Cl.: G01N 33/68

(54) **TEST PIECE FOR PROTEIN ASSAY AND PROCESS FOR PRODUCING THE SAME**

(30) Priority: 09.08.2002 JP 2002233468
(71) Applicant: Arkray, Inc., Kyoto-shi, Kyoto 601-8045 (JP)
(72) Inventor: Kosaka, Hideko, Arkray, Inc., Kyoto-shi, Kyoto 601-8045 (JP)
(74) Representative: Baldock, Sharon Claire
(86) International application number: PCT/JP2003/009889
(87) International publication number: WO 2004/015424

(57) **Abstract**

The present invention relates to a test piece for protein assay that is used for quantifying or semi-quantifying a protein and that contains an acidic pH indicator. This test piece for protein assay contains a surfactant as a sensitizer for increasing coloration sensitivity with respect to protein. A triphenylmethane-based indicator is used as the acidic pH indicator, for example. The present invention also provides a method for manufacturing a test piece for protein assay. With this method, a test piece for protein assay is manufactured by impregnating an absorbent carrier with an impregnant containing an acidic pH indicator and a sensitizer, and then drying this product. A surfactant is used as the sensitizer.

## Description

### TECHNICAL FIELD

This invention relates to a test piece for protein assay for assaying proteins present in protein-containing samples (body fluid such as blood and urine, or protein-containing beverages, or factory wastewater, etc.), and also relates to a method for manufacturing such a test piece.

### BACKGROUND ART

Assaying the protein in a biological sample is important in pathological diagnosis. For instance, the amount of serum albumin decreases in the case of diminished liver function, while the amount of protein in urine increases in the case of nephritis, nephrotic syndrome, lithiasis, tumors, other such kidney and urinary tract disorders, disorders of the circulatory system and disorders of central nervous system. Therefore, assaying albumin or other proteins can be an important clue in the diagnosis of these disorders.

A simple assay method featuring the use of a protein error indicator is known in the field of protein assay. With this assay method, tetrabromophenol blue (TBPB) is used, for instance, as the protein error indicator. As one example, urine test paper made with TBPB is widely used for primary screening purposes. TBPB changes from yellow to blue through the dissociation of phenolic hydroxyl groups at a pH of about 3 when a protein is present, and therefore can be used to detect protein.

However, test paper made using TBPB as the indicator has inadequate sensitivity with respect to the low protein concentrations of 10 to 20 mg/dL required for clinical use, and is therefore sometimes incapable of detecting protein accurately. For example, in a visual evaluation conducted by comparison with a color chart, the color is very similar between negative protein and trace protein, making it difficult to tell the two apart and hampering accurate evaluation. Meanwhile, when a urine test paper assay apparatus is used, the low sensitivity of TBPB often results in erroneous evaluation.

Consequently, there has been a need for a technique that would allow low concentrations of protein to be quantified at higher sensitivity.

### DISCLOSURE OF THE INVENTION

As a result of diligent research aimed at solving the above problems, the inventors arrived at the present invention upon discovering that low concentrations of protein can be assayed at high sensitivity if the test piece contains a surfactant.

Specifically, the test piece for protein assay provided by a first aspect of the present invention is a test piece for protein assay that is used for quantifying or semi-quantifying a protein and that contains an acidic pH indicator, containing a surfactant as a sensitizer for increasing coloration sensitivity to protein.

A second aspect of the present invention is a method for manufacturing a test piece for protein assay that is used for quantifying or semi-quantifying a protein, by impregnating an absorbent carrier with an impregnant containing an acidic pH indicator and a sensitizer, and then drying this product, wherein a surfactant is used as the sensitizer.

An example of an acidic pH indicator that can be used in the present invention is a triphenylmethane-based indicator. A typical example of a triphenylmethane-based indicator is expressed by the following Chemical Formula (1).

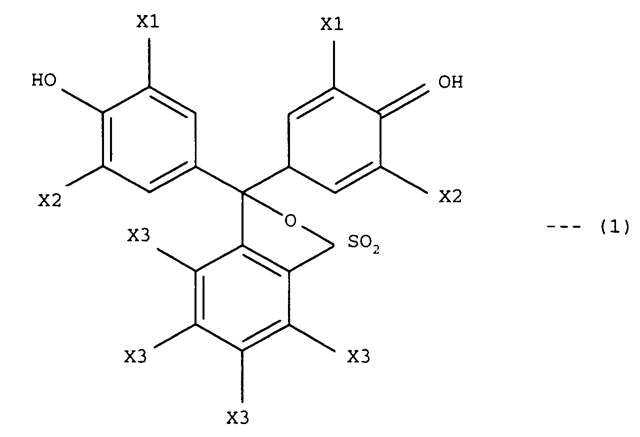

In Chemical Formula (1), X1 is a halogen, a nitro group, or a nitroso group, and X2 and X3 are the same or different halogens.

With the present invention, it is preferable to use the tetrabromophenol blue (TBPB) expressed by the following Chemical Formula (2) as the triphenylmethane-based indicator.

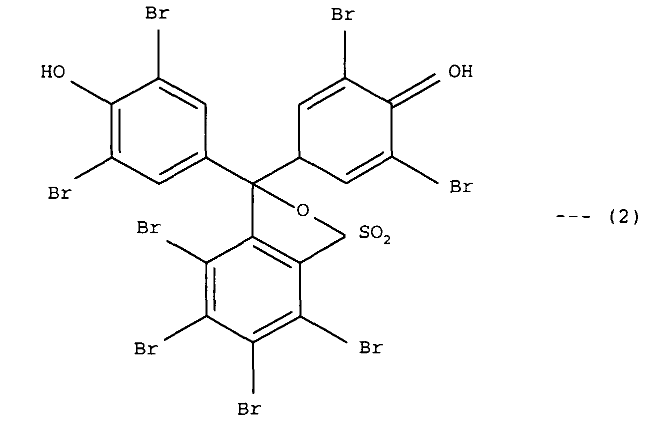

There are no particular restrictions on the concentration of the acidic pH indicator (such as TBPB) in the impregnant, but it is typically 0.1 to 5 mM, and preferably 0.3 to 1 mM.

It is preferable to use a cationic surfactant as the surfactant (sensitizer). In addition to this, or instead of this, a nonionic surfactant may be used.

A quaternary ammonium salt can be used, for example, as the cationic surfactant. This quaternary ammonium salt can be an alkyldimethylbenzylammonium, alkyltrimethylammonium salt, dialkyldimethylammonium salt, benzalkonium salt, imidazolium salt, or the like. An aliphatic amine salt cah also be used as the cationic surfactant.

The nonionic surfactant can be either an ether type, ether ester type, ester type, or nitrogen-containing type.

Examples of ether-type surfactants include polyoxyethylene alkyl ether, polyoxyethylene secondary alcohol ether, polyoxyethylene alkylphenyl ether, polyoxyethylene sterol ether, polyoxyethylene lanolin derivative, ethylene oxide derivative of alkylphenol formalin condensate, polyoxyethylene polyoxypropylene block polymer, and polyoxyethylene polyoxypropylene alkyl ether.

Examples of ether ester-type surfactants include polyoxyethylene glycerol fatty acid ester, polyoxyethylene castor oil, polyoxyethylene hydrogenated castor oil, polyoxyethylene sorbitan fatty acid ester, and polyoxyethylene sorbitol fatty acid ester.

Examples of ester-type surfactants include polyethylene glycol fatty acid ester, fatty acid monoglyceride, monoglycerol fatty acid ester, sorbitan fatty acid ester, propylene glycol fatty acid ester, and sucrose fatty acid ester.

Examples of nitrogen-containing surfactants include fatty acid alkanolamide, polyoxyethylene fatty acid amide, polyoxyethylenealkylamine, and alkylamine oxide.

The cationic surfactant used in the present invention is typically benzyltrimethylammonium bromide, hexadecyltrimethylammonium bromide, lauryltrimethylammonium bromide, or zephiramine, and the nonionic surfactant is typically polyethylene glycol. Naturally, a plurality of different surfactants may be used together, in which case it is preferable to use a combination of a cationic surfactant and a nonionic surfactant. Typically, a combination of benzyltrimethylammonium bromide, which is a cationic surfactant, and polyethylene glycol, which is a nonionic surfactant, is used.

There are no particular restrictions on the concentration of the sensitizer (surfactant) in the impregnant, but it is typically 0.01 to 5 wt%, and preferably 0.01 to 1 wt%.

A polycarbonate, polyvinyl alcohol, or other such polymer material may also be used in addition to the surfactant as a sensitizer.

The pH of the impregnant is set to be somewhat lower than the pKa of the acidic pH indicator. When TBPB is used as the acidic pH indicator, the pH of the impregnant is to be from 2.0 to 4.5, and preferably 2.0 to 3.5.

Any buffer can be used as long as it has a good buffering action at the pH of the impregnant (between 2.0 and 4.5, for instance) and does not impede the reaction between the acidic pH indicator and the protein. Examples of buffers that can be used include glycine buffer, citrate buffer, succinate buffer, malate buffer, and tartrate buffer. There are no particular restrictions on the concentration of the buffer in the impregnant, but it is typically from 0.1 to 1.5 M, and preferably 0.3 to 1 M.

A porous substance that contains no protein component can be used as the absorbent carrier, and can be used in the form of a sheet or film, for example. Examples of porous substances include paper-like materials, foams, woven materials, nonwoven materials, and knits. Examples of the material used to form the absorbent carrier include cotton, linen, cellulose, nitrocellulose, cellulose acetate, rock wool, glass fiber, silica fiber, carbon fiber, boron fiber, polyamide, aramid, polyvinyl alcohol, polyvinyl acetate, rayon, polyester, nylon, polyacrylic acid, polyacrylic ester, and polyolefin. There are no particular restrictions on the shape of the absorbent carrier, but it is generally rectangular (either short and wide or long and narrow), circular, or oval.

The test piece for protein assay of the present invention can be used directly as it is, or after first being bonded to a non-absorbent material.

The non-absorbent material is used in the form of a sheet or film, for example. Examples of the material used to form this non-absorbent material include polyethylene terephthalate, polyester, polypropylene, polyethylene, polyvinyl chloride, polyvinylidene chloride, and polystyrene.

### EXAMPLES

### [Example 1]

In this example, each test piece was impregnated with urine having an albumin concentration of 0.3 mg/dL (negative) or 15 mg/dL (positive), and the reflectance of the test piece was measured. The test pieces were formed by impregnating filter paper (3MMChr made by Whatman) with an impregnant, and then drying. The impregnant was produced by adding 0.2 wt% benzyltrimethylammonium bromide (a cationic surfactant) as a sensitizer to the base composition shown in Table 1. Reflectance was measured with a colorimeter at a measurement wavelength of 630 nm. The measurement results are given in Table 2.

### [Example 2]

In this example, reflectance was measured in the same manner as in Example 1, except that the impregnant was produced by adding hexadecyltrimethylammonium bromide (a cationic surfactant) as a sensitizer in an amount of 0.01 wt% to the base composition in Table 1. The measurement results are given in Table 2.

### [Example 3]

In this example, reflectance was measured in the same manner as in Example 1, except that the impregnant was produced by adding lauryltrimethylammonium bromide (a cationic surfactant) as a sensitizer in an amount of 0.01 wt% to the base composition in Table 1. The measurement results are given in Table 2.

### [Example 4]

In this example, reflectance was measured in the same manner as in Example 1, except that the impregnant was produced by adding polyethylene glycol (a nonionic surfactant) as a sensitizer in an amount of 0.5 wt% to the base composition in Table 1. The measurement results are given in Table 2.

### [Example 5]

In this example, reflectance was measured in the same manner as in Example 1, except that the impregnant was produced by adding benzyltrimethylammonium bromide (a cationic surfactant) in an amount of 0.2 wt% and polyethylene glycol (a nonionic surfactant) in an amount of 0.5 wt% as sensitizers to the base composition in Table 1. The measurement results are given in Table 2.

### [Example 6]

In this example, reflectance was measured in the same manner as in Example 1, except that the impregnant was produced by adding lauryltrimethylammonium bromide (a cationic surfactant) in an amount of 0.01 wt% and polyethylene glycol (a nonionic surfactant) in an amount of 0.5 wt% as sensitizers to the base composition in Table 1. The measurement results are given in Table 2.

### [Example 7]

In this example, reflectance was measured in the same manner as in Example 1, except that the impregnant was produced by adding zephiramine (a cationic surfactant) in an amount of 0.01 wt% and polyethylene glycol (a nonionic surfactant) in an amount of 0.5 wt% as sensitizers to the base composition in Table 1. The measurement results are given in Table 2.

### [Comparative Example 1]

In this comparative example, reflectance was measured in the same manner as in Example 1, except that the impregnant was the base composition itself (containing no surfactant (sensitizer)). The measurement results are given in Table 2.

**Table 1:**

| Base composition | | |
|---|---|---|
| Indicator | Buffer (pH 3.4) | Solvent |
| TBPB 0.5 mM | malate buffer 0.8 M | ethanol 30 wt% |

**Table 2:**

| Measurement results | | | | |
|---|---|---|---|---|
| | Sensitizer | Reflectance (%) | | Differential Δ(%) |
| | | 0.3 mg/dL (negative) | 15 mg/dL (positive) | |
| Ex. 1 | benzyltrimethylammonium bromide 0.2 wt% | 60.7 | 39.3 | 21.4 |
| Ex. 2 | hexadecyltrimethylammonium bromide 0.01 wt% | 60.4 | 40.6 | 19.8 |
| Ex. 3 | lauryltrimethylammonium bromide 0.01 wt% | 59.0 | 38.7 | 20.3 |
| Ex. 4 | polyethylene glycol 0.5 wt% | 57.0 | 40.4 | 16.6 |
| Ex. 5 | benzyltrimethylammonium bromide 0.2 wt% polyethylene glycol 0.5 wt% | 60.6 | 38.3 | 22.3 |
| Ex. 6 | lauryltrimethylammonium bromide 0.01 wt% polyethylene glycol 0.5 wt% | 59.1 | 35.9 | 23.2 |
| Ex. 7 | zephiramine 0.01 wt% polyethylene glycol 0.5 wt% | 61.2 | 41.5 | 19.8 |
| C. E. 1 | none | 60.7 | 48.9 | 11.8 |

As is clear from Table 2, reflectance at an albumin concentration of 15 mg/dL was lower when a surfactant was contained than when no surfactant was contained. Accordingly, the differential Δ (%) between negative (0.3 mg/dL) and positive (15 mg/dL) when a surfactant was contained was about twice that when no surfactant was contained. We can therefore conclude that when a surfactant is contained, sensitivity to albumin is higher, and albumin can be properly detected even when the albumin concentration is low (about 10 to 20 mg/dL). The albumin detection sensitivity was particularly high when a cationic surfactant was used alone and when it is used together with a nonionic surfactant. In this respect, a cationic surfactant can be considered to be preferable as a sensitizer.

The experiment results in these examples pertain to urine samples, but the present invention is not limited to urine, and can also be applied to the quantification of protein in any of various other samples containing protein, such as blood, protein-containing beverages, and factory wastewater.

## Claims

1. A test piece for protein assay, said test piece used for quantifying or semi-quantifying a protein and containing an acidic pH indicator,
wherein said test piece contains a surfactant as a sensitizer for increasing coloration sensitivity with respect to the protein.

2. The test piece for protein assay according to Claim 1, wherein the acidic pH indicator is a triphenylmethane-based indicator.

3. The test piece for protein assay according to Claim 2, wherein the triphenylmethane-based indicator is tetrabromophenol blue expressed by the following Chemical Formula (1).

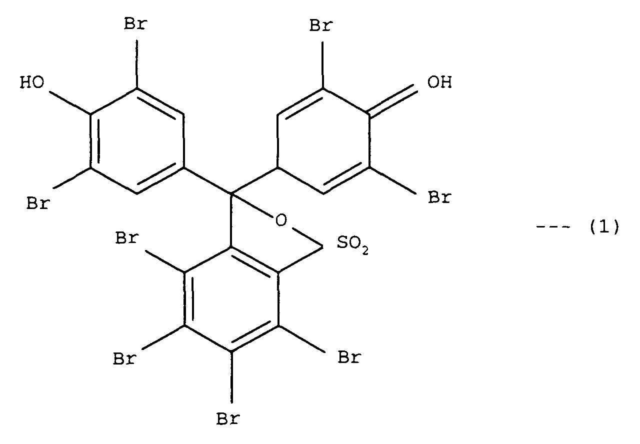

4. The test piece for protein assay according to Claim 1, containing a cationic surfactant as the sensitizer.

5. The test piece for protein assay according to Claim 4, wherein the cationic surfactant is at least one type selected from the group consisting of benzyltrimethylammonium bromide, hexadecyltrimethylammonium bromide, lauryltrimethylammonium bromide, and zephiramine.

6. The test piece for protein assay according to Claim 1, containing a nonionic surfactant as the sensitizer.

7. The test piece for protein assay according to Claim 6, wherein the nonionic surfactant is polyethylene glycol.

8. The test piece for protein assay according to Claim 1, wherein a combination of the cationic surfactant and nonionic surfactant is used as the sensitizer.

9. The test piece for protein assay according to Claim 8, wherein the cationic surfactant is benzyltrimethylammonium bromide and the nonionic surfactant is polyethylene glycol.

10. A method for manufacturing a test piece for protein assay that is used for quantifying or semi-quantifying a protein, the method comprising the steps of impregnating an absorbent carrier with an impregnant containing an acidic pH indicator and a sensitizer, and drying the carrier,
wherein a surfactant is used as the sensitizer.

11. The method for manufacturing a test piece for protein assay according to Claim 10, wherein the surfactant is at least one type selected from the group consisting of benzyltrimethylammonium bromide, hexadecyltrimethylammonium bromide, lauryltrimethylammonium bromide, zephiramine, and polyethylene glycol.

12. The method for manufacturing a test piece for protein assay according to Claim 11, wherein a combination of benzyltrimethylammonium bromide and polyethylene glycol is used as the surfactant.

13. The method for manufacturing a test piece for protein assay according to Claim 10, wherein the amount of surfactant contained in the impregnant is set between 0.01 and 5 wt%.

14. The method for manufacturing a test piece for protein assay according to Claim 13, wherein the amount of surfactant contained in the impregnant is set between 0.01 and 1 wt%.

15. The method for manufacturing a test piece for protein assay according to Claim 10, wherein the tetrabromophenol blue expressed by the following Chemical Formula (2) is used as the acidic pH indicator.

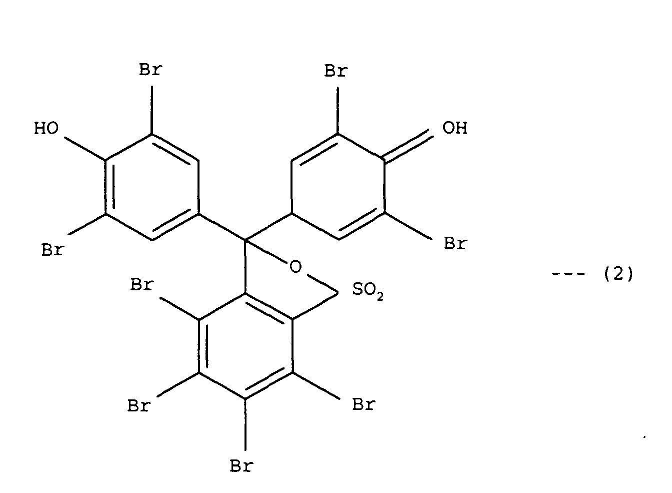

16. The method for manufacturing a test piece for protein assay according to Claim 10, wherein the concentration of the acidic pH indicator in the impregnant is set between 0.1 and 5 mM.

17. The method for manufacturing a test piece for protein assay according to Claim 16, wherein the pH of the impregnant is set at or below the pKa of the acidic pH indicator.

18. The method for manufacturing a test piece for protein assay according to Claim 17, wherein the pH of the impregnant is set between 2.0 and 4.5.
